# EUROPEAN PATENT APPLICATION

(11) **EP 2 839 849 A1**
(43) Date of publication of application: **25.02.2015**
(21) Application number: 13778464.1
(22) Date of filing: 19.04.2013
(51) Int. Cl.: A61L 27/00, A61F 2/28

(54) **BIOMATERIAL COATED WITH HAp/Col COMPOSITE**

(30) Priority: 19.04.2012 JP 2012096056
(71) Applicant: National Institute for Materials Science, Tsukuba-shi, Ibaraki 305-0047 (JP); National University Corporation Tokyo Medical and Dental University, Bunkyo-ku Tokyo 113-8510 (JP)
(72) Inventor: KIKUCHI, Masanori, Tsukuba-shi Ibaraki 305-0047 (JP); TAKAKUDA, Kazuo, Tokyo 113-8510 (JP); MORIYAMA, Keiji, Tokyo 113-8510 (JP); SUZUKI, Shoichi, Tokyo 113-8510 (JP); UEZONO, Masayoshi, Tokyo 113-8510 (JP)
(74) Representative: Calamita, Roberto
(86) International application number: PCT/JP2013/061666
(87) International publication number: WO 2013/157638

(57) **Abstract**

A biomaterial in which the surface of a metal base is coated with a coating agent containing a composite of hydroxyapatite and collagen in which the c-axis of hydroxyapatite in the composite is oriented along collagen fibers is used.

## Description

### Technical Field

0001 This invention relates to a biomaterial which is implanted in a living body.

### Background Art

0002 As described in PTL 1 for example, for biomaterials such as an artificial tooth root implant and an artificial bone implant, titanium or an alloy material thereof with excellent biocompatibility is used as the constituting metal material, and it is known that the biocompatibility becomes excellent by coating the surface of the metal base with hydroxyapatite.

0003 Here, it is known that bone of vertebrates is a composite of an inorganic substance, hydroxyapatite (HAp), and an organic substance, collagen, that HAp forms in bone a unique nanocomposite structure in which HAp is oriented along collagen fibers in its c-axis direction and that this structure achieves the unique mechanical properties of bone. Accordingly, when an artificial biomaterial is produced for example, it is not possible to obtain the structure and characteristics similar to those of bone by merely combining hydroxyapatite (HAp) and collagen.

0004 Therefore, an artificial biomaterial is required to have effects of fusing with a bone tissue and actively promoting bone regeneration in addition to the affinity to a living body. That is, bone conductivity and bioactivity for being gradually absorbed after being applied in a living body, incorporated into the bone regeneration cycle and replaced with the bone of the host are required. In this regard, hydroxyapatite (HAp), which is an inorganic substance, is excellent in the affinity to bone and collagen (Col), which is an organic substance, is effective in promoting the cell adhesion property and cell differentiation. Therefore, a HAp/Col composite is expected to have properties of an excellent artificial biomaterial.

0005 From the above background, various studies have been conducted to develop an organic/inorganic composite biomaterial which is closer to bone using hydroxyapatite and collagen.

0006 PTL 2, for example, discloses a method for producing an apatite/organic substance composite in which a mixture of a collagen solution and phosphoric acid is gradually added to a suspension of calcium hydroxide and thus a formed material having a Young's modulus similar to that of bone is obtained. PTL 3 discloses a method for producing an organic/inorganic orientational composite material in which an aqueous phosphoric acid solution containing collagen and an aqueous solution containing calcium salt are simultaneously dropped into a reaction container while the pH and the temperature during the reaction are controlled and a formed material similar to bone is obtained by pressure-forming the precipitates generated. PTL 4 discloses a technique to improve the apatite formation on collagen surface using organic acids.

0007 Moreover, PTL 5 discloses an organic/inorganic composite biomaterial which contains a composite of hydroxyapatite and collagen having an average fiber length of 60 µm or more and has a microporous structure in which the c-axis of hydroxyapatite is oriented along collagen fibers. An artificial bone implant, a joint prosthesis, cement for the tendon and bone, a dental implant and the like are disclosed as the methods of utilizing the organic/inorganic composite biomaterial.

### Citation List

### Patent Literature

0008
[PTL 1] JP-A-2006-314760
[PTL 2] JP-A-7-101708
[PTL 3] JP-A-11-199209
[PTL 4] JP-A-2000-5298
[PTL 5] JP-A-2003-190271

### Summary of Invention

### Technical Problem

0009 A biomaterial coated with hydroxyapatite such as the one described in PTL 1, however, has problems in that it takes time to join the implanted biomaterial and bone and further improvement is necessary in order to put it into practice.

0010 In addition, PTLs 2 to 5 merely suggest the effectiveness of a bone-like composite containing hydroxyapatite and collagen, and no specific study was conducted, for example regarding the method and problems for promoting joining of a metal biomaterial implanted in a living body and bone.

0011 This invention was made in view of the above circumstances and aims to provide a biomaterial achieving excellent osteosynthesis which can promote joining of an implanted biomaterial and bone.

### Solution to Problem

0012 In order to solve the above problems, the biomaterial of this invention is a biomaterial in which the surface of a metal base is coated with a coating agent containing a composite of hydroxyapatite and collagen and is characterized in that the c-axis of hydroxyapatite in the composite is oriented along collagen fibers.

0013 In the biomaterial, the ratio by weight of hydroxyapatite and collagen in the composite is preferably 3:2 to 9:1.

0014 The biomaterial is preferably an implant material or an onplant material for oral surgery, orthopedic surgery, brain surgery, dentistry, ophthalmology or otolaryngology.

0015 The coating agent for promoting osteosynthesis for the biomaterial of this invention is characterized by containing a composite of hydroxyapatite and collagen in which the c-axis of hydroxyapatite is oriented along collagen fibers.

0016 The method for producing the biomaterial of this invention is characterized by containing a step of coating the surface of a metal base with the coating agent for promoting osteosynthesis.

### Advantageous Effects of Invention

0017 In this invention, the capsulation of the biomaterial is prevented and joining of the biomaterial and bone can be promoted by using a coating agent containing a HAp/Col composite in which the c-axis of hydroxyapatite is oriented along collagen fibers.

### Brief Description of Drawings

0018
[Fig. 1] Fig. 1 is a figure in which the surface of a wire coated with the HAp/Col composite was observed with a scanning electron microscope.
[Fig. 2] Fig. 2 is a figure in which the surface of a wire coated with the HAp/Col composite was observed with a scanning electron microscope.
[Fig. 3] Fig. 3 is a figure showing the results of histomorphometry based on an image of a tissue slice using image analysis software.
[Fig. 4] Fig. 4 is a figure showing the results of histomorphometry based on an image of a tissue slice using image analysis software.
[Fig. 5] Fig. 5 is a schematic figure showing the summary of the shear strength test.
[Fig. 6] Fig. 6 includes figures in which tissue slices prepared from samples collected from (A) group without coating, (B) group with hydroxyapatite (HAp) coating and (C) group with HAp/Col composite coating were observed with a light microscope. The pictures in the lower row are enlarged views of the pictures in the upper row.
[Fig. 7] Fig. 7 includes figures in which tissue slices prepared from samples collected from (A) group without coating, (B) group with hydroxyapatite (HAp) coating and (C) group with HAp/Col composite coating were observed with a light microscope. The pictures in the lower row are enlarged views of the pictures in the upper row.
[Fig. 8] Fig.8 is a chart showing the measurement results of bone contact ratios of samples collected from (A) group without coating, (B) group with hydroxyapatite (HAp) coating and (C) group with HAp/Col composite coating.
[Fig. 9] Fig.9 is a chart showing the measurement results of new bone heights of samples collected from (A) group without coating, (B) group with hydroxyapatite (HAp) coating and (C) group with HAp/Col composite coating.
[Fig. 10] Fig.10 is a chart showing the results of the shear strength test of samples collected from (A) group without coating, (B) group with hydroxyapatite (HAp) coating and (C) group with HAp/Col composite coating.
[Fig. 11] Fig.11 is a chart showing the results of the shear strength test of samples collected from (A) group without coating, (B) group with hydroxyapatite (HAp) coating and (C) group with HAp/Col composite coating.
[Fig. 12] Fig.12 includes figures showing the observation results with µCT before and after the shear strength test of samples collected from (A) group without coating, (B) group with hydroxyapatite (HAp) coating and (C) group with HAp/Col composite coating.

### Description of Embodiments

0019 While the present inventors were studying the joining of a biomaterial and bone, the inventors found that joining of a biomaterial and bone delays because the implanted biomaterial is covered with a fibrous coating (capsulation) and osteogenic cells are prevented from penetrating into the surface of the biomaterial. Furthermore, on the assumption that joining of a biomaterial and bone can be promoted if the capsulation of the biomaterial can be prevented until osteogenic cells have proliferated and migrated, the inventors completed this invention.

0020 The biomaterial of this invention is explained in detail below.

0021 In the biomaterial of this invention, the surface of a metal base is coated with a coating agent containing a composite of hydroxyapatite (HAp) and collagen (Col) (a HAp/Col composite).

0022 Regarding the coating agent containing a composite of hydroxyapatite (HAp) and collagen (Col), which is applied on the surface of the biomaterial of this invention, for example the fibrous composite of hydroxyapatite (HAp) and collagen (Col) and the production method thereof which are described in PTL 5 (JP-A-2003-190271) can be considered.

0023 Hydroxyapatite is a compound with a general composition of Ca₅(PO₄)₃OH and includes a group of compounds called calcium phosphate such as CaHPO₄, Ca₃(PO₄)₂, Ca₄O(PO₄)₂, Ca₁₀(PO₄)₆(OH)₂, CaP₄O₁₁, Ca(PO₃)₂, Ca₂P₂O₇ and Ca(H₂PO₄)₂·H₂O by the nonstoichiometric properties of its reaction. In addition, hydroxyapatite is basically composed of a compound represented by a formula Ca₅(PO₄)₃OH or Ca₁₀(PO₄)₆(OH)₂ and a part of the Ca component may be substituted with one or more selected from Sr, Ba, MG, Fe, Al, Y, La, Na, K, H and the like. Furthermore, a part of the (PO₄) component may be substituted with one or more selected from VO₄, BO₃, SO₄, CO₃, SiO₄ and the like. Moreover, a part of the (OH) component may be substituted with one or more selected from F, Cl, O, CO₃ and the like. Some of these components may be defects. Because a part of the PO₄ and OH components of apatite in bone is generally replaced with CO₃, CO₃ may be incorporated from the air and may partially replace the components (about 0 to 10% by mass) during the production of the composite biomaterial.

0024 In this regard, hydroxyapatite may be in the form of isomorphic solid solution, substitutional solid solution or interstitial solid solution as well as general microcrystalline, non-crystalline or crystalline form and may contain a nonstoichiometric deficiency. Moreover, in the "hydroxyapatite", the atomic ratio of calcium and phosphorus (Ca/P) is preferably within the range of 1.3 to 1.8 and is more preferably within 1.5 to 1.7 in particular. This is because, when the atomic ratio is within the range of 1.3 to 1.8, the composition and crystalline structure of apatite in the product (a calcium phosphate compound) are similar to the composition and structure of apatite in bone of vertebrates and thus the biocompatibility and bioabsorbability improve.

0025 It is now known that about 20 kinds of collagen with different molecular species exist in tissues of various animals including fish as well as mammals, and the generic term thereof is "collagen". Regarding the collagen used in this invention, the kind, tissue part, age and the like of the animal providing the starting material are not particularly limited and any type can be used. In general, collagen obtained from the skin, bone, cartilage, tendon, organ and the like of mammals (such as cow, pig, horse, rabbit and mouse) or birds (such as chicken) is used. Furthermore, a collagen-like protein obtained from the skin, bone, cartilage, fin, scale, organ and the like of fish (such as cod, flounder, plaice, salmon, trout, tuna, mackerel, sea bream, sardine and shark) may be used as the starting material. Alternatively, collagen obtained by genetic transformation, not by extraction from an animal tissue, may be used.

0026 Here, the molecular species of collagen that is the most abundant and most well studied is type I collagen, and in many cases, mere collagen generally refers to type I collagen. The molecular species of collagen used in this invention is not particularly limited but it is preferable to contain type I collagen as the main component. Furthermore, regarding collagen, amino acid residues of the collagen protein may be appropriately chemically modified for example by acetylation, succinylation, maleylation, phthalation, benzoylation, esterification, amidation, guanidination or the like.

0027 Examples of the method for preparing collage are methods of extracting collagen from the above starting materials with a neutral buffer solution or a dilute acid such as hydrochloric acid, acetic acid and citric acid. Collagen prepared by the former method is called neutral salt-soluble collagen and collagen prepared by the latter method is called acid-soluble collagen. However, the amount of collagen extracted is low in both methods and most of the collagen remains as insoluble collagen. As the method for solubilizing insoluble collagen, an enzyme-solubilizing method and alkali-solubilizing method are known. Collagen prepared by the former method is called enzyme-solubilized collagen and collagen prepared by the latter method is called alkali-solubilized collagen, which can be both solubilized as molecular collagen in yield of about 100%.

0028 The method for preparing the collagen used in this invention (extraction type) is not particularly limited but it is preferable to use monomeric (monomolecular) collagen because the strength of the composite becomes insufficient due to the steric hindrance if the molecular weight of solubilized collagen is large. In particular, enzyme-solubilized collagen and alkali-solubilized collagen include a large amount of monomeric components and the non-helical regions (telopeptides) having most of the antigenicity of collagen are selectively degraded and removed during the preparation step, and thus these types of collagen are preferable. In this regard, collagen from which non-helical regions are degraded and removed is called atelocollagen.

0029 Here, isoionic points are different between enzyme-solubilized collagen and alkali-solubilized collagen. An isoionic point is the pH at which positive and negative charges derived from a dissociable group inherent to the protein molecule balance with each other, and in the case of collagen, it is known that solubilized collagen becomes fibrous when the pH becomes close to the pH region of the isoionic point. In general, the isoionic point of enzyme-solubilized collagen is pH 8 to 9 and the isoionic point of alkali-solubilized collagen is pH 4 to 5. In this invention, it is more preferable to use enzyme-solubilized collagen whose fibrillization advances in a reaction container in which the pH is kept at 7 to 11 and which is easily self-assembled. Furthermore, examples of the enzyme for solubilizing are pepsin, trypsin, chymotrypsin, papain and Pronase and pepsin and Pronase are preferably used because treatment after the enzyme reaction is easy.

0030 Furthermore, it is preferable that hydroxyapatite and collagen are oriented in a self-assembled way and form a fibrous composite similar to bone. In this regard, the term "self-assembly" generally means that "homologous or heterologous atoms, molecules, fine particles or the like assemble through noncovalent interaction and form a unique tissue ("Seikagaku Jiten (Dictionary of Biochemistry)", Tokyo Kagaku Dojin Co., Ltd.)." However, in this invention in particular, the term means a microporous structure in which calcium phosphate (hydroxyapatite: HAp) having an apatite structure is oriented along collagen fibers in a manner unique to bone, that is, in such a way that the c-axis of HAp (the crystallographic main axis (vertical axis)) is oriented along collagen fibers.

0031 The metal base is processed to have a form appropriate for an implant material, an onplant material or the like for oral surgery, orthopedic surgery, brain surgery, dentistry, ophthalmology or otolaryngology for example, and specific examples of the form are a wire, a pin, a screw, a nail, a mesh and a plate. The materials of the metal base are not particularly limited and known metal materials can be appropriately selected depending on the use of the biomaterial. In view of the biocompatibility, preferable examples are titanium and a titanium alloy. Furthermore, the metal materials may be those in which the surface has been subjected to treatment such as etching, sandblast, mechanical processing, laser processing, particle-baking and the like depending on the use.

0032 The fibrous composite of hydroxyapatite and collagen can be produced using at least three components, namely collagen, a phosphate and a calcium salt, as the starting materials. In this regard, although they are not "a salt" in a precise sense, the phosphate includes phosphoric acid and the calcium salt includes calcium hydroxide in this invention.

0033 The phosphoric acid sources of an aqueous phosphate solution which is used in this invention are disodium hydrogenphosphate, sodium dihydrogenphosphate, dipotassium hydrogenphosphate, potassium dihydrogenphosphate, phosphoric acid and the like. The aqueous phosphate solution is subjected to the reaction after dissolving the collagen described above.

0034 Examples of the calcium source of an aqueous calcium salt solution which is used in this invention are calcium carbonate, calcium acetate and calcium hydroxide. The aqueous calcium salt solution may be a suspension as long as it is in a homogeneous state, and for example, a suspension of calcium hydroxide obtained by baking calcium carbonate, pulverizing it in a mortar or the like to obtain calcium hydroxide and then adding water can be preferably used.

0035 When the fibrous composite containing hydroxyapatite and collagen is produced, it is possible to simultaneously drop the aqueous calcium salt solution and the aqueous phosphate solution containing collagen into a reaction container. Here, the term "simultaneously" means not only the embodiment of simultaneous dropping in a precise sense but also the embodiment of dropping small amounts (about 0.01 to 5 ml) one after the other. Both solutions may be dropped continuously or intermittently as long as they are dropped simultaneously. Specifically, it is possible to add an adequate amount of purified water in the reaction container in advance. The amount of purified water is not particularly limited but it is preferable that the amount is approximately the same as the amount of the aqueous calcium salt solution used.

0036 When the fibrous composite of hydroxyapatite and collagen is produced, it is particularly preferable that the concentration of calcium ions in the reaction container is kept to be 3.75 mM or less and the concentration of phosphoric ions is kept to be 2.25 mM or less. If the concentrations of calcium ions and phosphoric ions exceed the above ranges, preferable self-assembly of the composite may be hindered. This is thought to be because spontaneous nucleation occurs if the concentrations of ions circulating in the reaction container exceed the concentrations in the body fluid. In this regard, when the concentration of calcium ions and the concentration of phosphoric ions are kept to be 2.5 mM and 1.5 mM or less, respectively, a composite having an average fiber length of 1 mm or more can be obtained, which is more preferable.

0037 Moreover, hydroxyapatite and collagen generated in the reaction container exist preferably in the ratio by weight of 3:2 to 9:1 and more preferably 70:30 to 85:15. This is because it is important for self-assembly that the ratio by weight of hydroxyapatite and collagen after the ideal reaction is as close to the composition of bone (75:25) as possible.

0038 When the fibrous composite of hydroxyapatite and collagen is produced, the concentration of calcium ions and the concentration of phosphoric ions in the reaction container can be maintained in the desired ranges by controlling the sending rates of the aqueous calcium salt solution and the aqueous phosphate solution containing collagen and/or the concentrations of the aqueous calcium salt solution and the aqueous phosphate solution containing collagen at the starting stage.

0039 Here, the term "concentrations at the starting stage" mean the concentrations of the components (the aqueous calcium salt solution, the aqueous phosphate solution containing collagen and the like) which have been individually prepared, before introducing them into the reaction container. Further, the "sending rates" mean the fluid volumes per unit time of the reaction solutions sent into the reaction container. The sending rates can be easily adjusted for example by using a commercially available tube pump.

0040 In this regard, the sending rates of the aqueous calcium salt solution and the aqueous phosphate solution are each adjusted in such a way that dropping of both solutions end almost at the same time (at least within a lag of 10 minutes).

0041 In a preferable embodiment of this invention, in the case where the ratio by weight of hydroxyapatite and collagen generated is kept to 70:30 to 85:15, the concentration of the aqueous calcium salt solution at the starting state, with the average sending rate of the aqueous calcium salt solution set to 5 to 25 mM/min, is 400 mM or less and preferably within the range of 50 to 200 mM. Furthermore, the concentration of the aqueous phosphoric acid solution containing collagen is 120 mM or less and preferably within the range of 15 to 96 mM. In this regard, the term "average sending rate" means the average value of the fluid volume sent to the reaction container per minute, considering the on-off operation of the pump and the like.

0042 The ratio of the aqueous phosphoric acid solution containing collagen and the aqueous calcium salt solution is preferably within the range of 3:1 to 1:3. This is because, if the amount of the aqueous phosphoric acid solution containing collagen is low, the strength deteriorates due to the composition with excess calcium; while calcium lacks and the Young's modulus decreases, which sometimes result in the decrease in the strength, if the amount of the aqueous solution containing calcium salt is low (please refer to JP-A-11-199209).

0043 In this invention, it is preferable to drop the solutions in such a way that the pH of the reaction solution is within the range of 7 to 11 and the variation thereof is 1 or smaller. More preferably, the pH is within the range of 7 to 9 and the variation is 0.5 or smaller. This is because native collagen precipitates at pH 7 to 11 due to the isoelectric point, resulting in the regeneration of fibers, and calcium phosphate is likely to precipitate in this pH range, and thus self-assembly of calcium phosphate and collagen advances in this pH range. If the pH exceeds 11, water molecules hydrate around collagen molecules and water molecules do not easily separate. Thus, the water content of the composite increases, the self-assembly is prevented and the strength may decrease. On the other hand, if the pH is less than 7, both calcium phosphate and collagen do not easily precipitate. Furthermore, if the variation exceeds 1, the nucleation of calcium phosphate on collagen is disturbed and the self-assembly deteriorates (Kikuchi et. al., Biomaterials 22, (2000) p1705-1711)).

0044 When the fibrous composite of hydroxyapatite and collagen is produced, an easy way to preferably control the pH is to use a pH controller. A pH controller has a means for measuring the pH of the reaction solution and a means for controlling the amount of both solutions dropped, and controls the amounts of both solutions based on the pH values of both solutions in such a way that the pH is kept within a certain range (for example, ±0.3) relative to the pH set as the predetermined value (for example 10). An example of the pH controller is a pH controller produced by NISSIN Corporation. In this regard, it is preferable to conduct the reaction while constantly stirring both solutions and the reaction solution in order to keep the pH of the reaction solution even.

0045 Furthermore, when the fibrous composite of hydroxyapatite and collagen is produced, it is preferable to keep the temperature of the reaction solution at 35°C to 40°C. This is because, at the temperature within this range, it is expected that the composite is formed under the similar condition as in a living body.

0046 The average fiber length of the fibrous composite of hydroxyapatite and collagen is preferably 60 µm or more. Practically, the average fiber length is more preferably 1 mm to 7 mm and further preferably 3 mm to 7 mm. In this regard, the "average fiber length" is an average value of the lengths of the fibers consisting of the composite and is measured with a particular device (for example, RapidVue manufactured by Beckman-Colter, Inc. and the like) or measured visually.

0047 Thus produced fibrous composite of hydroxyapatite and collagen can be processed into fine fibers or particles, for example through pulverizing treatment, forming treatment and the like, before applying the fibrous composite on the surface of the metal base. Hereinafter, in this invention, including the processed composites of a fibrous form or fine fibrous form having a fiber length within the above range, of a particle form and the like, the composite is described as "the composite of hydroxyapatite and collagen (HAp/Col composite)".

0048 As the method for coating the surface of the metal base with the composite of hydroxyapatite and collagen (HAp/Col composite), known coating methods such as immersion coating can be used. For example, in the case of immersion coating, by immersing the metal base in a solution to which the composite of hydroxyapatite and collagen (HAp/Col composite) has been added, the surface of the metal base can be coated with the composite of hydroxyapatite and collagen (HAp/Col composite). Furthermore, the solvent to which the composite of hydroxyapatite and collagen (HAp/Col composite) is added is not particularly limited and for example, purified water, known simulated body fluids (for example, Li P. Biomimetic nano-apatite coating capable of promoting bone ingrowth. J Biomed Mater Res 2003;66A:79) and the like can be used. In addition, the ratio of the solvent and the solute (HAp/Col composite) is not particularly limited but, as a rough indication, examples of the composition is approximately solvent:solute=5:1 to 5:4 and preferably solvent:solute=3:2 (ratio by weight). When an aqueous solvent is used, it is possible to conduct dehydration treatment using ethanol and the like before air-drying, vacuum-drying or vacuum freeze-drying, in order to evenly and rapidly dry the water-containing coated layer soon after coating. In addition, in the case of immersion coating, the number of immersions of the metal base can be adjusted considering the state of the coated HAp/Col composite, the coating thickness and the like. The coating thickness can be appropriately designed within the range of around 1 µm to 80 µm for example, considering the use of the biomaterial and the like.

0049 Furthermore, in order to control the degradation speed while securing the stability of the coated layer, by introducing cross-linking into collagen in the coated layer of the composite of hydroxyapatite and collagen (HAp/Col composite), it is possible to control the biodegradation speed. Furthermore, in order to increase the cross-linked cites, it is possible to add a small amount (1 to 100 mol% relative to the collagen amount in the composite) of collagen or polysaccharides.

0050 Cross-linking may be by any method of thermal cross-linking, chemical cross-linking using a cross-linking agent or a condensing agent, physical cross-linking using gamma-ray, ultraviolet ray, thermal dehydration, electron beam and the like. In the case of thermal cross-linking, collagen chains are cross-linked and the coating can be made strong by vacuum-heating the material in which the coated layer is sufficiently dry, for example at around 120°C for 24 hours. Examples of the chemical cross-linking agent are aldehyde cross-linking agents such as glutaraldehyde and formaldehyde; isocyanate cross-linking agents such as hexamethylene diisocyanate; carbodide cross-linking agents such as 1-ethyl-3-(3-dimethylaminopropyl)carbodiimide hydrochloride; polyepoxy cross-linking agents such as ethylene glycol diethyl ether: and transglutaminase. The amount of the cross-linking agent is preferably about 10 µmol to 10 mmol based on 1 g of collagen.

0051 Cross-linked cites may be at any cites of collagen but it is preferable to cross-link carboxyl group and hydroxyl group, carboxyl group and ε-amino group, or ε-amino groups in particular. Furthermore, it is preferable that at least 1 % or more of the reactive functional groups are cross-linked and it is more preferable that 5% or more are cross-linked.

0052 When the composite of hydroxyapatite and collagen (HAp/Col composite) is produced, it is possible to further add other components in addition to the calcium salt, phosphate and collage, which are the essential components, as long as the objects and effects of this invention are not impaired. Examples of such components are inorganic salts of Sr, Mg, CO₃ and the like, organic substances such as citric acid and phospholipids, and pharmaceutical preparations such as bone morphogenetic protein.

0053 It is generally considered that titanium and hydroxyapatite join bone, however, osteosynthesis may not occur or osteosynthesis may take some time depending on the location and condition of the implantation. The present inventors have obtained knowledge that the problems regarding the osteosynthesis of a biomaterial arise because the implanted biomaterial is covered with a fibrous coating (fibroblasts) (capsulation) and osteogenic cells are prevented from penetrating into the surface of the biomaterial. That is, in the case of the conventional biomaterials coated with hydroxyapatite for example, it is thought that the capsulation occurs and the osteosynthesis of the biomaterials and bone is prevented as a result of the proliferation of fibroblasts around the surface and the formation of a matrix at an early stage after the implantation.

0054 On the contrary, in the biomaterial of this invention, the surface of the metal base is coated with the coating agent containing the composite in which the c-axis of hydroxyapatite is oriented along collagen fibers. Accordingly, when the biomaterial is implanted in a living body (for example at an intraosseous or subperiosteal part), the HAp/Col composite is absorbed at an adequate rate while the HAp/Col composite is phagocytized and thus the capsulation of the biomaterial is prevented. Specifically, with the biomaterial of this invention, the absorption of the HAp/Col composite begins soon after the implantation, but the HAp/Col composite remains until after about one week and the HAp/Col composite is absorbed over one to two weeks after the implantation. Accordingly, with the biomaterial of this invention, the capsulation is prevented, and the metal base of the biomaterial is exposed and the osteosynthesis occurs on the surface of the metal base at the point at which osteogenic (osteoblastic) cells have started to proliferate and migrate. Thus, it is possible to promote joining of the biomaterial and bone. Thus, in this invention, the coating agent containing the HAp/Col composite has a significant effect to promote the osteosynthesis.

0055 In this regard, the method for implanting the biomaterial of this invention and the method for fixing it are not particularly limited.

0056 Examples of the biomaterial of this invention include an implant material, an onplant material or the like for oral surgery, orthopedic surgery, brain surgery, dentistry, ophthalmology or otolaryngology. Specifically, examples of the biomaterial of this invention include those in which the surface of the metal base having a form such as a wire, a pin, a screw, a nail, a mesh or a plate is coated with the coating agent containing the HAp/Col composite and they can be used for various medical uses.

0057 The biomaterial of this invention is not limited to the above embodiments.

### Examples

0058 This invention is explained further in detail below by Examples; however, this invention is not limited to these Examples.

### <1> Production of biomaterial

### (1) Preliminary treatment of metal base

A pure titanium wire was used as the metal base. Specifically, a pure titanium wire having a diameter of 0.5 mm was cut into 12.0 mm pieces and the wire surface was made rough with emery paper. Furthermore, the wires were subjected to ultrasonic cleaning each for 30 minutes in a neutral detergent, purified water, acetone, ethanol and purified water in this order.

### (2) Coating of wire surface

In order to subject to the animal experiment, the preliminarily-treated wires were divided into three types, namely, (A) without coating, (B) hydroxyapatite (HAp) coating and (C) HAp/Col composite coating. Specifically, wires treated by the following methods were used.

0059

### (A) Without coating

The wires that were treated in (1) above were used as they were.

0060

### (B) Hydroxyapatite (HAp) coating

Referring to the report of Li P. et al. (Li P. Biomimetic nano-apatite coating capable of promoting bone ingrowth. J Biomed Mater Res. 2003;66A:79.), a simulated body fluid was prepared and operation to immerse the wires in the simulated body fluid under the condition of 45°C for three days was repeated for three times for coating.

0061

### (C) HAp/Col composite coating

In accordance with the method described in PTL 5, a coating agent containing the HAp/Col composite was prepared. Specifically, in a reaction container in which the temperature was set to 40°C and the pH was set to 9, starting materials prepared in such a way that the ratio by mass of HAp and Col was 80/20 (a suspension of calcium hydroxide and a phosphoric acid solution containing type I atelocollagen derived from pig skin) were reacted by simultaneous dropping method and a fibrous HAp/Col composite having an average fiber length of 60 µm was obtained. In this HAp/Col composite, the c-axis of hydroxyapatite was oriented along collagen fibers. By adding 800 µL of purified water to 0.1 g of the obtained fibrous HAp/Col composite and pulverizing the HAp/Col composite with a pestle for grinding cells, a sol suspension in which the HAp/Col composite was evenly dispersed was obtained. Then, the wires were hold with tweezers at room temperature, immersed in the suspension containing the HAp/Col composite, moved up and down for two to three times in the solution and then taken out.

0062 After taking out the wires from the HAp/Col suspension, the wires were immersed in 100% ethanol for 20 to 30 seconds to dehydrate the coated layer. The wires were taken out and air-dried and ethanol and remaining water were vaporized. In this regard, after air-drying, if coating of the HAp/Col composite on the wire surface is insufficient, the above operation can be repeated. After air-drying the wire surface, the wires were heated under vacuum for 12 hours at 140°C with a vacuum oven to thermally cross-link the collagen chains in the coated layer.

0063

### <2> Observation of wire surface

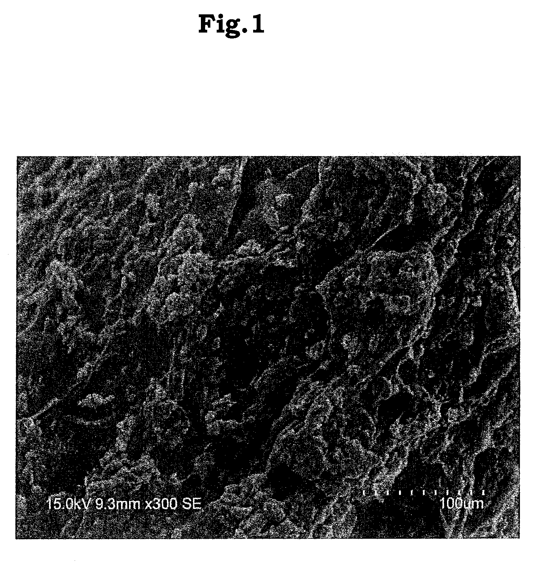

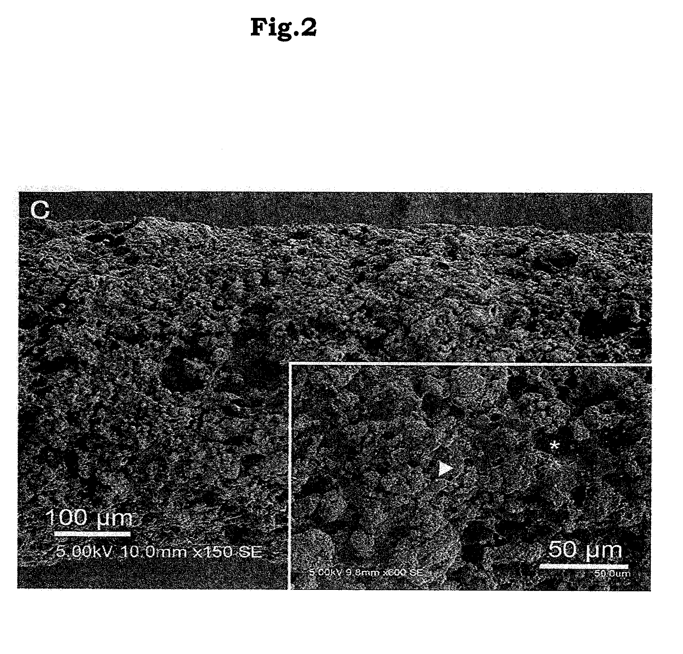

The observation results of the wire surface coated with the HAp/Col composite with a scanning electron microscope are shown in Fig.1 and Fig. 2. As shown in Fig. 1, it is confirmed that the wire surface is coated with the HAp/Col composite having a microporous structure. Also in the slightly enlarged view of Fig.2, it is confirmed that the wire surface is coated with the HAp/Col composite having a microporous structure. In addition, as shown in the greatly enlarged view of Fig. 2 (the lower right figure), it is confirmed that the wire surface is coated with particles (the part indicated with the arrow in Fig. 2) of about 10 µm which are thought to be derived from HAp and a pattern structure (the part indicated with * in the greatly enlarged view of Fig. 2) which is thought to be derived from Col. It is also confirmed that the particles which are thought to be derived from HAp have a porous structure of nanometer order.

0064

### <3> Animal experiment

The three types of wire above, namely, (A) without coating, (B) hydroxyapatite (HAp) coating and (C) HAp/Col composite coating, were each implanted in the subperiosteal part of the skull of 12-week-old SD male rats. Hereinafter, the rat groups are referred to (A) group without coating, (B) group with hydroxyapatite (HAp) coating and (C) group with HAp/Col composite coating.

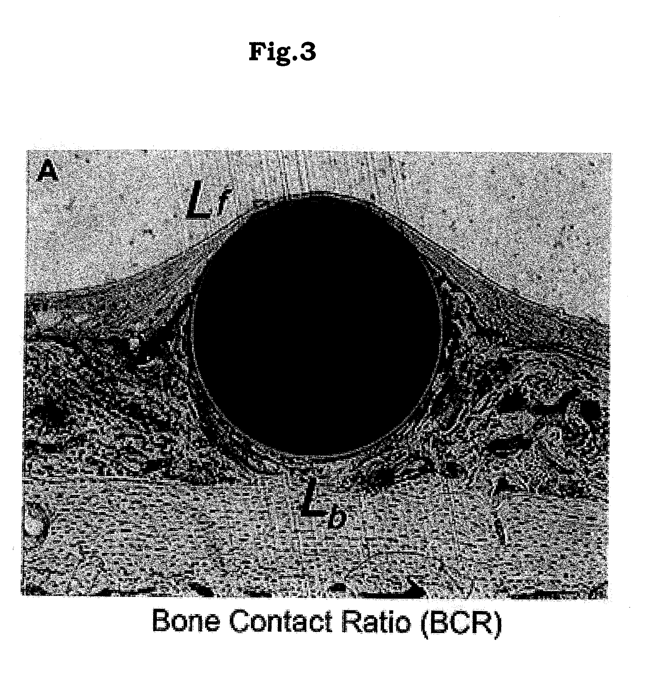

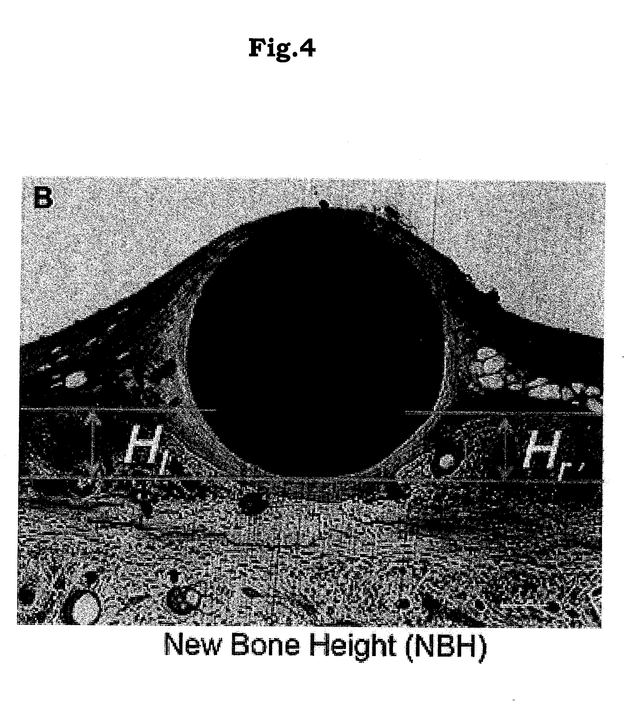

0065 After a four-week implant period, samples together with skulls were collected, fixed over one week at room temperature using 70% ethanol, embedded with a MMA resin embedding kit, cut into slices with a thickness of about 10 µm with a microtome for hard tissues, stained with Villanueva staining solution and then observed histologically with a light microscope. In addition, based on tissue slice images, using image analysis software, histomorphometry was conducted. First, the length of the part in contact with bone (L_{b}) and the length of the entire circumference of the wire (L_{f}) were measured and the length of the part in contact with bone (L_{b}) was divided by the length of the entire circumference of the wire (L_{f}) to calculate the bone contact ratio (BCR) (the bone contact ratio = the length of the part in contact with bone (L_{b}) / the length of the entire circumference of the wire (L_{f})) (Fig. 3). Furthermore, the heights of the new bone at both sides from the wire bottoms (H_{I}, H_{R}) were measured and the average value thereof was calculated as the new bone height (NBH) (Fig. 4). 0066

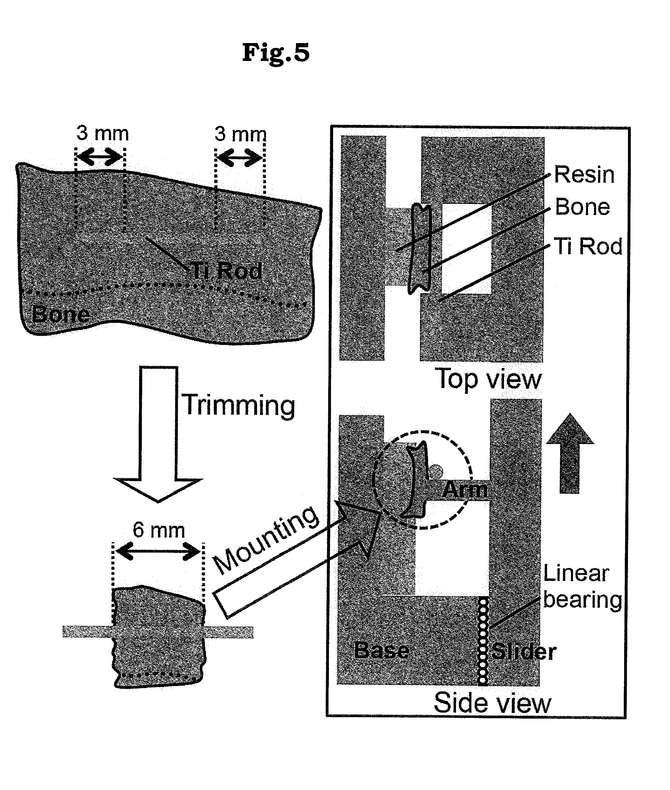

Moreover, the joining strengths of the samples were also evaluated by the shear strength test (Fig. 5). Specifically, the wires and the skulls were collected together, the samples were kept in physiological saline at 4°C and within one hour, the shear strength test was conducted. Those which were trimmed with a diamond disk in such a way that both edges of the wire were exposed from the skull, with the central part of the wire of within 6.0 mm maintained, were used as the samples. A sample was set to a jig produced for the mechanical test in such a way that the wire was located perpendicularly to the direction of movement of the jig, a load was applied in one direction to both edges of the wire (1.0 mm/min), and thus the shear strength was measured. In order to observe the samples before and after the shear strength test, µCT images were taken and observed.

In this regard, in the histomorphometry and the shear strength test, the sample number of each group was 5 and the measurement results were statistically tested using multiple rank-sum test of Wilcoxon with significance levels adjusted with Holm's method.

0067

### <4> Results

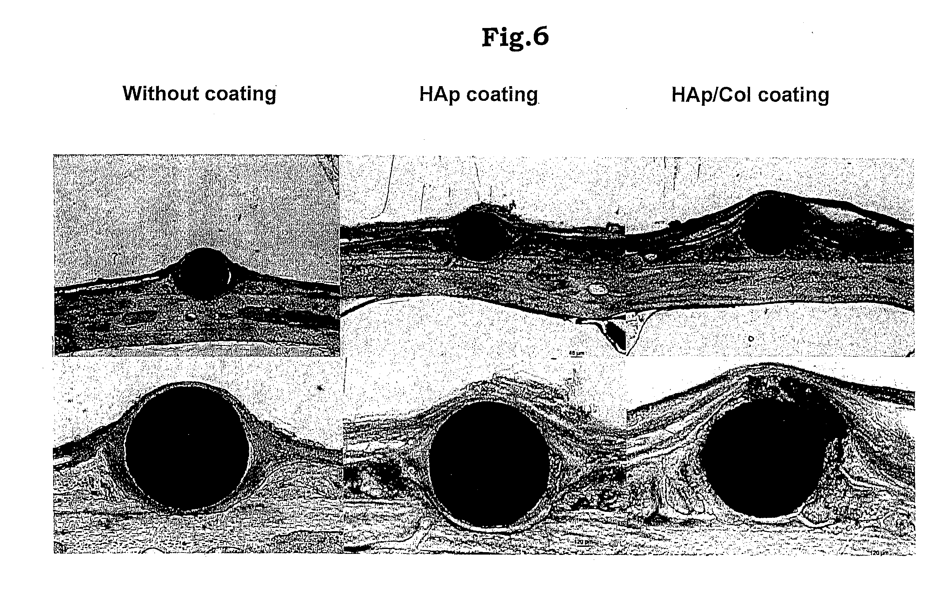

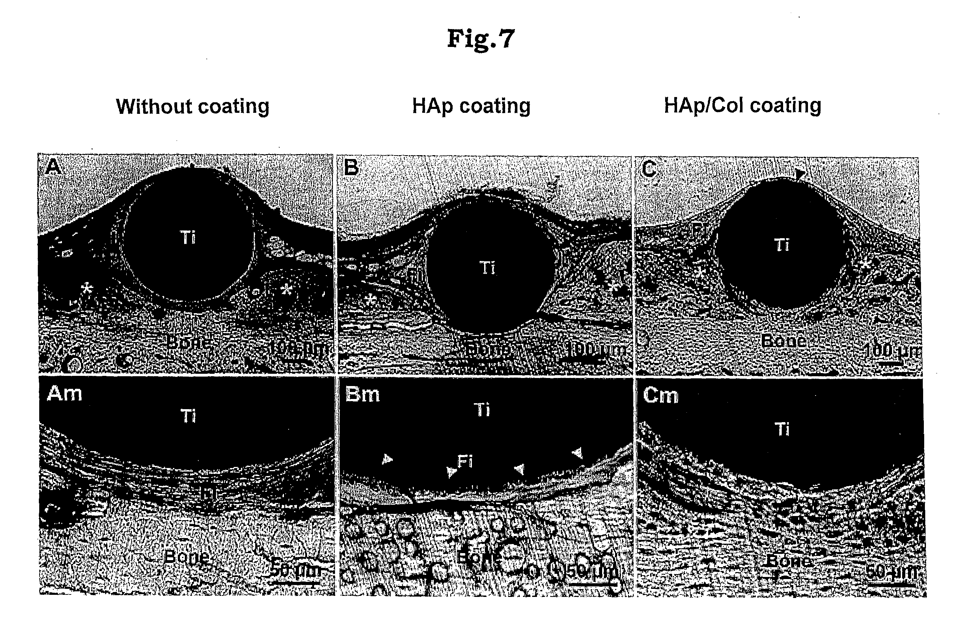

The results of histological observation are shown in Fig. 6 and Fig. 7.

0068 As shown in Fig. 6 and Fig. 7, in (A) group without coating, the wires (Ti) were capsuled with fibrous tissues (Fi); in the example shown in Fig. 6, the contact of the wire and the new bone was scarcely observed (the contact ratio with bone: 6.5%) and in the example shown in Fig. 7, the contact of the wire and the new bone was not observed (the contact ratio with bone: 0%). In (B) group with hydroxyapatite (HAp) coating, although joining with bone was partially observed (the parts indicated with the arrows in Fig. 7), most part of the wire surface was similarly capsuled and excellent osteosynthesis was not observed (in the example shown in Fig. 6, the contact ratio with bone: 11.6%, and in the example shown in Fig. 7, the contact ratio with bone: 21.1±25.9%). In (C) group with HAp/Col composite coating, the capsulation of the wire surface was prevented and direct joining of the wire and the new bone (parts indicated with * in Fig. 7) without fibrous tissues was observed (in the example shown in Fig. 6, the contact ratio with bone: 52.4%, and in the example shown in Fig. 7, the contact ratio with bone: 62.2%±7.9%).

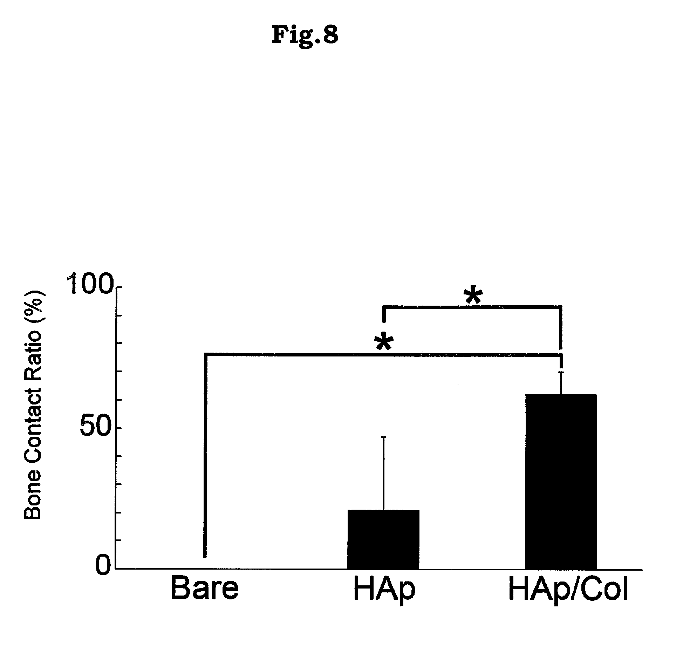

The results of the bone contact ratios observed in the examples shown in Fig. 7 are shown in Fig. 8. A significant difference was observed between (A) group without coating and (C) group with HAp/Col composite coating (P<0.05). Furthermore, a significant difference was observed between (B) group with HAp coating and (C) group with HAp/Col composite coating (P<0.05).

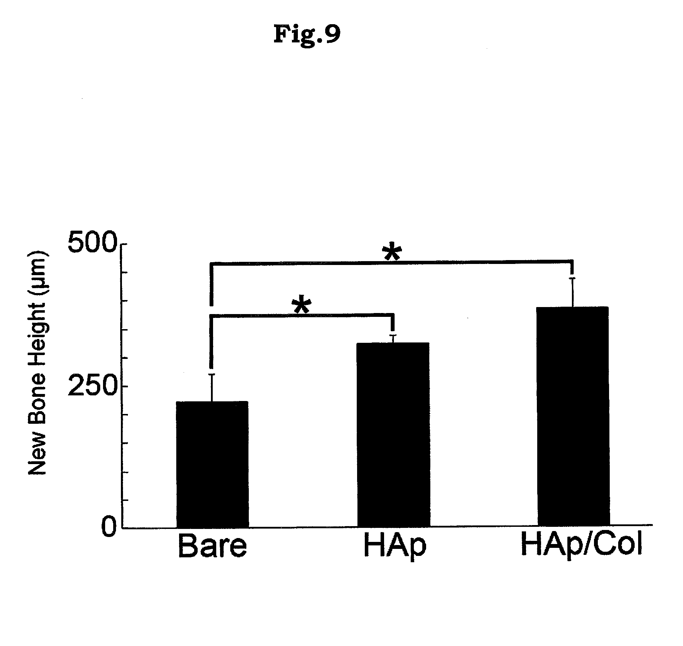

The results of the new bone heights are shown in Fig. 9. A significant difference was observed between (A) group without coating and (B) group with HAp coating (P<0.05). Furthermore, a significant difference was observed between (A) group without coating and (C) group with HAp/Col composite coating (P<0.05).

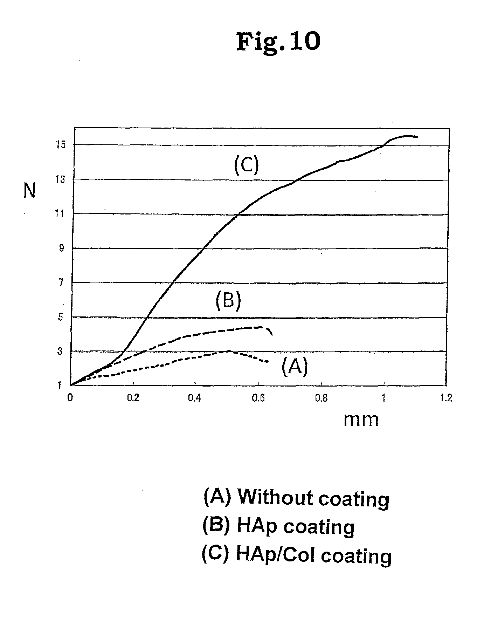

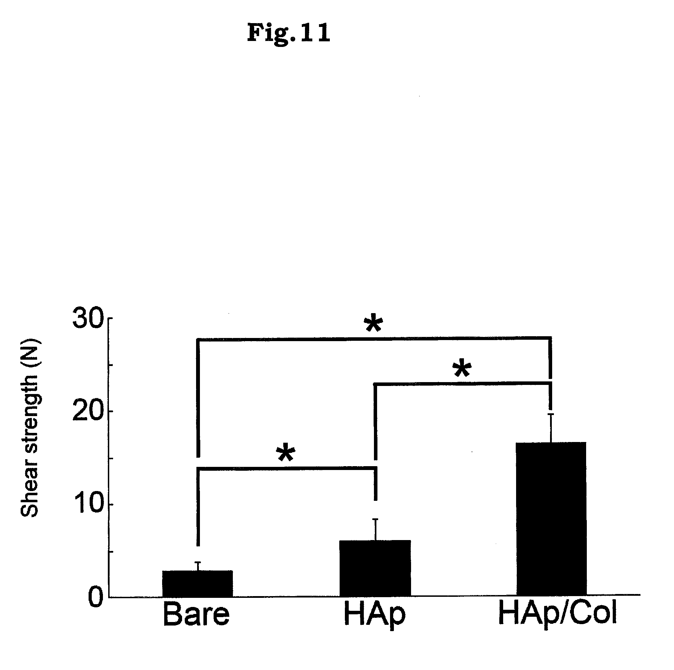

0069 The results of the shear strength test are shown in Fig. 10 and Fig. 11.

0070 The largest strengths measured in the shear strength test were: in (A) group without coating, 3.46 N in the example shown in Fig. 10 and 2.8±0.9 N in the example shown in Fig. 11; in (B) group with hydroxyapatite (HAp) coating, 4.31 N in the example shown in Fig. 10 and 6.0±2.2 N in the example shown in Fig. 11; and in (C) group with HAp/Col composite coating, 14.8 N in the example shown in Fig. 10 and 16.4±3.0 N in the example shown in Fig. 11. It was confirmed that the joining force with bone of (C) group with HAp/Col composite coating was significantly large.

In addition, as shown in Fig. 11, (C) group with HAp/Col composite coating showed the largest shear strength and significant differences were found between the groups (P<0.05).

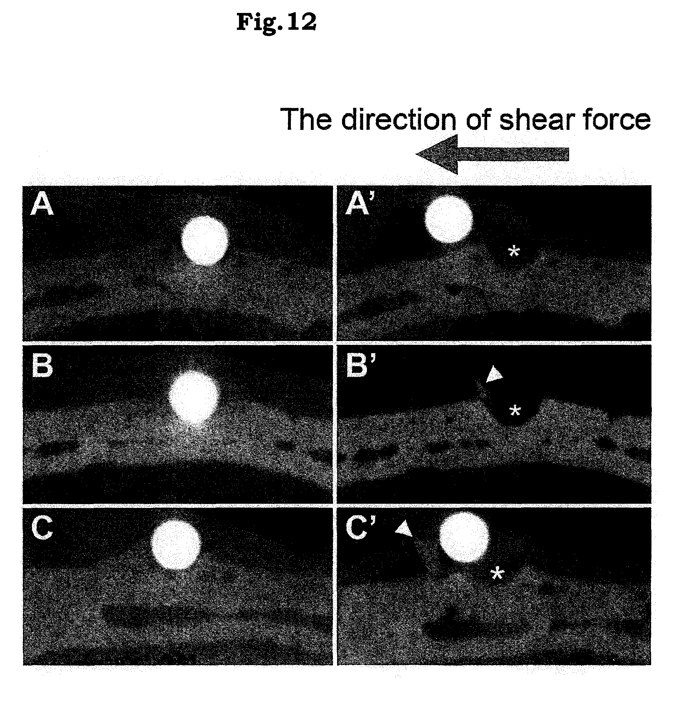

The results of the observation with µCT before and after the shear strength test are shown in Fig. 12.

In the samples before the shear strength test (A to C), new bone was observed around the wires in all the groups. In the samples after the shear strength test (A' to C'), although bone fracture was not observed in (A') group without coating, bone fractures (the parts indicated with the arrows in the figures) were observed in (B') group with HAp coating and (C') group with HAp/Col composite coating. Furthermore, a larger bone fracture piece was observed in (C) group with HAp/Col composite coating than in (B) group with HAp coating. That is, it was confirmed that the joining force of bone and wire was significantly large in (C) group with HAp/Col composite coating.

0071 From the above results, it was confirmed that the capsulation of the biomaterial can be prevented and joining of the biomaterial (metal base) and bone can be promoted by coating the surface of the metal base with the coating agent containing the HAp/Col composite.

## Claims

1. A biomaterial in which the surface of a metal base is coated with a coating agent containing a composite of hydroxyapatite and collagen, wherein the c-axis of hydroxyapatite in the composite is oriented along collagen fibers.

2. The biomaterial of claim 1, wherein the ratio by weight of hydroxyapatite and collagen in the composite is 3:2 to 9:1.

3. The biomaterial of claim 1 or 2, wherein the biomaterial is an implant material or an onplant material for oral surgery, orthopedic surgery, brain surgery, dentistry, ophthalmology or otolaryngology.

4. A coating agent for promoting osteosynthesis for a biomaterial which contains a composite of hydroxyapatite and collagen in which the c-axis of hydroxyapatite is oriented along collagen fibers.

5. A method for producing a biomaterial which contains a step of coating the surface of a metal base with the coating agent for promoting osteosynthesis of claim 4.
